# EUROPEAN PATENT SPECIFICATION

(11) **EP 4 590 177 B1**
(45) Date of publication and mention of the grant of the patent: **25.11.2026**
(21) Application number: 23782302.6
(22) Date of filing: 22.09.2023
(51) Int. Cl.: A61B 5/00, A61B 5/0205, A61B 5/024, A61B 5/0507, A61B 5/08, A61B 5/16, A61B 5/18, G08B 21/06

(54) **REAL-TIME SLEEP PREDICTION BASED ON STATISTICAL ANALYSIS OF A REDUCED SET OF BIOMETRIC DATA**
ECHTZEIT-SCHLAFVORHERSAGE AUF DER BASIS VON STATISTISCHER ANALYSE AUS EINEM REDUZIERTEN SATZ BIOMETRISCHER DATEN
PRÉDICTION DE SOMMEIL EN TEMPS RÉEL BASÉE SUR UNE ANALYSE STATISTIQUE D'UN ENSEMBLE RÉDUIT DE DONNÉES BIOMÉTRIQUES

(30) Priority: 22.09.2022 EP 22197077; 21.09.2023 IT 202300019470
(43) Date of publication of application: 30.07.2025
(73) Proprietor: Sleep Advice Technologies S.r.l., 10129 Torino (IT); Politecnico Di Torino, 10129 Torino (IT)
(72) Inventor: GROPPO, Riccardo, 10129 Torino (IT); VIOLANTE, Massimo, 10129 Torino (IT); PUGLIESE, Luigi, 10129 Torino (IT)
(74) Representative: Studio Torta S.p.A.
(86) International application number: PCT/IB2023/059385
(87) International publication number: WO 2024/062439

(56) References cited:
- US-A1- 2018 214 089
- US-A1- 2022 015 654

## Description

### Cross-Reference to Related Patent Applications

This patent application claims priority of European patent application No. 22197077.5 filed on 22.09.2022 and of Italian patent application No. 102023000019470 filed on 21.09.2022.

### Technical Field of the Invention

The present invention generally relates to real-time detection and/or prediction of one or more behavioural states and/or transitions among Awake (W), Drowsiness (D) and Sleep (S) phases of a subject, in particular the sleep onset of a subject through statistical analysis applied on a reduced set of biometric data.

Thus, the present invention aims at warning and to provide means to interact with a subject before loss of cognitive performance of the latter, thereby significantly reducing the possibility of a traumatic event, for example a car accident.

### State of the Art

As is known, non-invasive recognition of different behavioural phases (Awake W, Drowsiness D, Sleep S) of individuals is a problem that concerns some of the main areas of modern life, such as public health and safety in transportation and working environments, with important consequences in socio-economic terms and with important impulses to research and development area.

The wake-sleep transition is difficult to define since it is a process instead of an on-off phenomenon or a precise moment where such transition happens. In particular, falling asleep can be defined as a transition phase between wakefulness and sleep, *i.e.* the time interval between the moment in which a subject is predisposed to sleep and the moment in which the subject actually sleeps.

While the definition of wakefulness and sleep is relatively simple also from an operational point of view, defining the phase of falling asleep is more difficult, since it includes fragments of wakefulness and sleep continuously alternating with each other, thereby determining a lack of a "moment" where falling asleep occurs but rather the presence of a period (Ogilvie 2001) in which numerous variables (namely, neurophysiological and behavioural) fluctuate until the onset of sleep. The difficulty in defining the wake-sleep transition also derives from the adoption, in known studies of falling asleep, of the Standard System of Classification of sleep phases (*i.e.* Rechtschaffen & Kales), which uses the same parameters (namely for epochs of 30 seconds) to analyse such heterogeneous periods, *e.g.* falling asleep that lasts a few minutes and sleep that lasts several hours.

Thus, ultimately, the process of falling asleep (also defined sleep onset, SO) may be considered as multi-dimensional, entailing, *e.g.*, subjective, behavioural and physiological dimensions.

To systematically evaluate stages of drowsiness and facilitate the development of automatic early drowsiness detection systems, the Applicant has noticed that a precise measurement scale for drowsiness levels is necessary; on this regard, several known methods have been proposed, some of which are described below.

It is foremost noted that one of the most widely used scales in the literature is the Karolinska Sleepiness Scale (KSS), which is a scale that measures the subjective levels of sleepiness at a particular time during the day; specifically, KSS is a nine-point scale that measures drowsiness through verbal descriptions of drivers, ranging from level (1) *"Extremely alert"* to level (9) *"Very sleepy, great effort to keep alert".* Subjective sleepiness rating scales, such as the KSS, are quick, easy, and low-cost methods. The Applicant also notes that it is important to underline that, as self-evaluations, they can be influenced both by the surrounding environment and by the emotional state of the subject; very often individuals also tend to overestimate or underestimate their sleepiness; therefore, the results obtained from said methods are often not superimposed on objective measures.

In order to detect drowsiness, the following known different measures have been implemented:
- Image based measures: some drowsiness signs are visible and can be recorded by cameras or visual sensors, in particular by recording the driver's facial expressions and movements, especially the head movements. Generally, such systems are non-intrusive, non-invasive and cost-effective, as they require only a camera to collect the needed data, and they are able to provide details on the subject's state of sleepiness at a very advanced stage, when his cognitive state is no longer able to carry out its activity; however, the system's performance is significantly affected in cases where it is difficult to track facial data due to obstacles and they cannot cover the large population of people who fall asleep with their eyes open, especially people with Obstructive Sleep Apnea Syndrome, OSAS;
- Biological-Based Measures: many biological signals, such as brain activity, heart rate, breathing rate, pulse rate and body temperature signals, have been used to detect the driver's drowsiness. These biological signals, also known as physiological measures, are proven to be more accurate and reliable for detecting drowsiness; in particular, the accuracy is due to their ability to capture early biological changes that may appear, in the case of drowsiness, thus alerting the driver before any physical drowsiness signs appear. Several activities are aimed at developing cost effective and the least intrusive, possibly contactless, sensors able to provide accurate measurement of the required biometric parameters;
- Vehicle-Based Measures: this method depends on tracing and analysing driving patterns, the latter forming a unique driving pattern. Thus, the driving patterns of a drowsy driver can be distinguished from those of an alert driver. However, since it is an indirect way of detecting drowsiness, such solution is neither accurate nor fast enough to regain the consciousness level of the driver; and
- Hybrid-Based Measures: a hybrid drowsiness detection system exploits a combination of image-, biological, and vehicle-based measures to extract drowsiness features, with the aim of producing a more robust, accurate and reliable drowsiness detection system.

A further known system for detecting and predicting transitions between awake, drowsiness and sleep phases is disclosed in the international patent application WO 2020/043855 A1 and US 2022/015654 A1, wherein full photopletismography (PPG) signal is used.

### Object and Summary of the Invention

The Applicant notes that, in modern wearable devices, the PPG signal is even more often acquired and internally processed by dedicated hardware circuitries which optimise the cost/performance feature; the Applicant notes that the same holds also for contactless sensors (*e.g.,* RF, RADAR) where only processed biometric data are available.

The object of the present invention is to provide a new approach through statistical analysis applied on a reduced set of biometric data to predict the sleep onset in real-time on a wide range of modern, both contact and contactless, edge devices.

According to the present invention, a software and electronic processing resources to real-time detect and/or predict one or more behavioural states and/or transitions among Awake (W), Drowsiness (D) and Sleep (S) phases of a subject are provided, as claimed in the appended claims.

### Brief Description of the Drawings

Figure 1 schematically shows a system comprising electronic processing resources according to the present invention.
Figure 2 schematically shows a software according to the present invention and implementing a four KSS level classification.
Figure 3 shows a block diagram of the software according to the present invention.
Figure 4 schematically show an architectural mapping in an integrated and distributed manner according to the present invention.
Figure 5 shows a block diagram of an integrated type architecture according to the present invention.
Figure 6 shows a block diagram of a distributed type architecture according to the present invention.
Figure 7 shows a distributed type architecture applied to a first use case according to the present invention.
Figure 8 shows a distributed type architecture applied to a second use case according to the present invention.
Figure 9 schematically show an architectural mapping in a distributed and remote manner according to the present invention.

### Detailed Description of Preferred Embodiments of the Invention

The present invention will now be described in detail with reference to the accompanying drawings in order to allow a skilled person to implement it and use it. Various modifications to the described embodiments will be readily apparent to those of skill in the art and the general principles described may be applied to other embodiments and applications without however departing from the protective scope of the present invention as defined in the appended claims. Therefore, the present invention should not be regarded as limited to the embodiments described and illustrated herein but should be allowed the broadest protection scope consistent with the features described in the appended claims.

Unless otherwise defined, all technical and scientific terms used herein have the same meaning commonly understood by one of ordinary skill in the art to which the invention belongs. In case of conflict, the present specification, including the definitions provided, will control. Furthermore, the examples are provided for illustrative purposes only and as such should not be considered limiting.

In particular, the block diagrams included in the attached figures and described below are not to be understood as a representation of the structural features, i.e. constructional limitations, but must be understood as a representation of functional features, i.e. intrinsic properties of the devices defined by the effects obtained, that is to say functional restrictions, which can be implemented in different ways, so as to protect the functionalities thereof (operational capability).

In order to facilitate the understanding of the embodiments described herein, reference will be made to some specific embodiments and a specific language will be used to describe the same. The terminology used herein is used for the purpose of describing particular embodiments only and is not intended to limit the scope of the present invention.

In particular, as also described in the following, the present invention aims at extending the possibility of accurately predicting the onset of sleep to the large variety of systems in which the complete PPG signal is not accessible or available without satisfactory quality, thus being able to satisfy a greater number of application fields.

Furthermore, the present invention is based on a simplified model of the autonomic nervous system (ANS) that controls the behaviour of the cardio-respiratory system (CRS); in particular, the CRS behaviour is monitored in real-time by an algorithm for sleep prediction. This algorithm processes a small number of physiological parameters, which are measured either through contact-based sensing devices (*e.g*., custom or commercial off-the-shelf smart watches) or through contactless sensing devices (*e.g.,* RF sensors such as RADAR or camera-based sensors), to derive a number of statistical parameters. Based on the variability of such statistical parameters over time, the present invention allows to classify the person's drowsiness state and it identifies the sleep onset.

Thus, as also described below, the present invention introduces a methodology to process physiological data coming from either contact or contactless sensing solution and derive a multilevel classification of the drowsiness of a person; according to the present invention, reference will be made, without it being limiting, to a four-levels scale derived from the KSS, in particular a four step KSS. In any case, the present invention may be applied to any newly-defined drowsiness assessment scales. In this way, the present invention allows to provide both a more accurate scale in the drowsy region, which is by definition dealing with a non-binary process, and accurately identify whether a subject is alert (*i.e.* the subject is perfectly able to perform the task) or sleepy (the subject is no longer able to perform the task in a controlled manner).

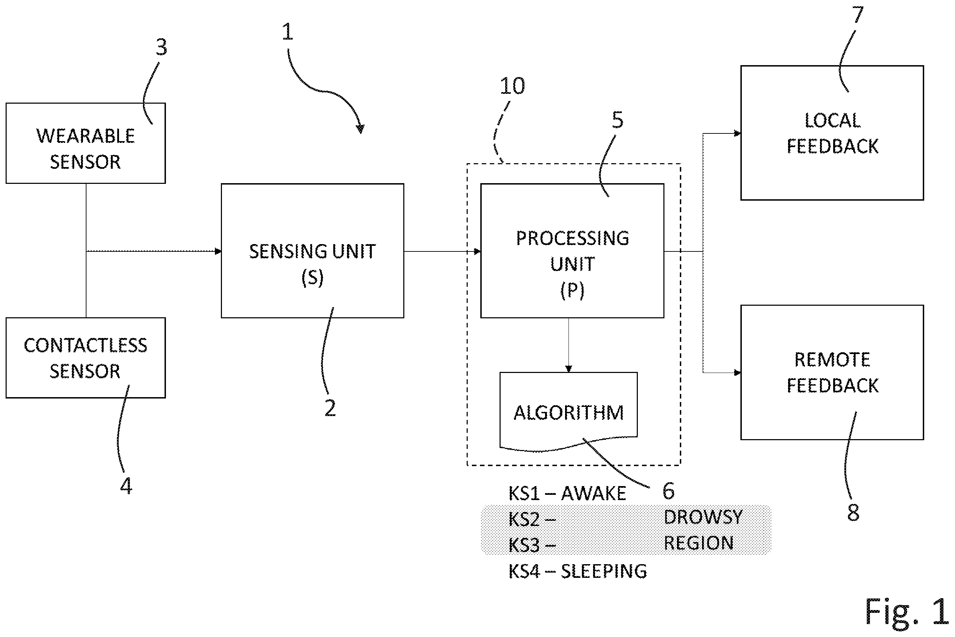

Figure 1 shows a block diagram of a system **1** comprising:
- a sensing unit **2** configured to communicate with a sensory system, here comprising either a wearable sensor **3** (*e.g.* a smartwatch) and/or a contactless sensor **4** (*e.g.* a RADAR, a camera), to receive biometric signals of a subject;
- electronic computing resources **10,** here comprising a processing unit **5,** configured to communicate with the sensing unit **2** to receive the biometric signals and to store, load and execute, when in use, a software or algorithm **6** therein to output data relative to the behavioural state and/or transitions between behavioural states of the subject on the basis of the biometric signals; and
- a feedback unit, here comprising either a local feedback unit **7** and/or a remote feedback unit **8,** configured to communicate with the electronic computing resources **10** and to receive the output generated by the latter to provide feedback to external resources, e.g. further electronic devices external to the system **1.**

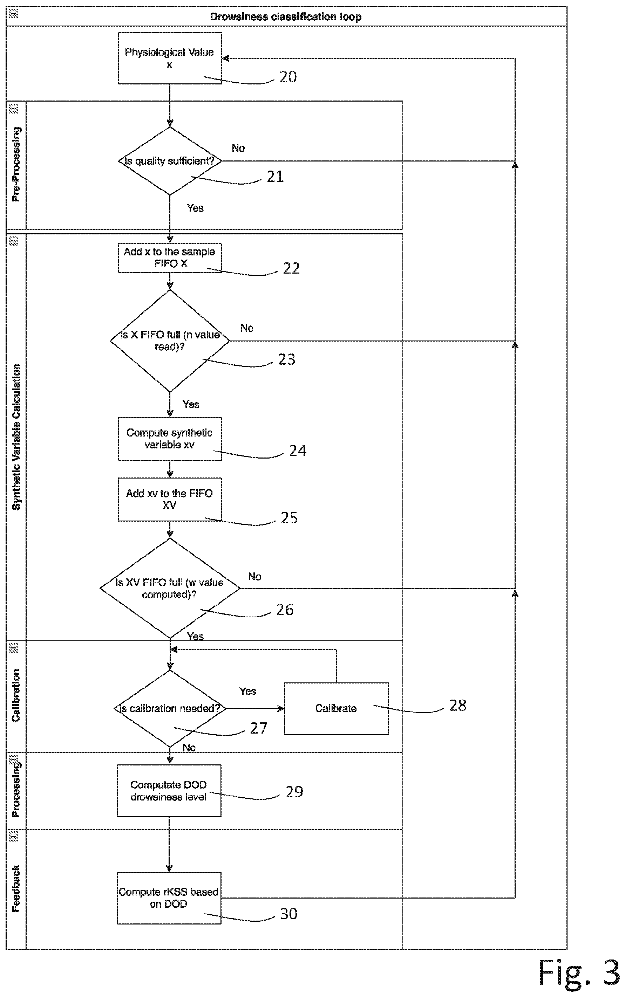

In particular, the software **6** is designed to cause, when executed, the electronic processing resources **10** to become configured to real-time detect and/or predict one or more behavioural states and/or transitions among Awake (W), Drowsiness (D) and Sleep (S) phases of the subject. In further detail, referring to Figure 3, the software **6** is designed to cause, when executed, the electronic processing resources **10** to become configured to:
- receive (block **20)** a biometric signal of the subject, here from the sensory system through the sensing unit **2;**
- process (blocks **21-29)** the received biometric signal to classify it into one of different classes associated with the one or more behavioural states and/or transitions among Awake (W), Drowsiness (D) and Sleep (S) phases of a subject; and
- detect and/or predict (block **30)** a behavioural state and/or a transition among awake (W), Drowsiness (D), and Sleep (S) phases of the subject based on the classified biometric signal.

In particular, the software is designed to cause, when executed, the electronic processing resources **10** to become configured to:
- compute (block **24)** at least a first one synthetic quantity for and based on the received biometric signal;
- compute (block **29)** at least one threshold Th for the received biometric signal based on the at least first one synthetic quantity computed therefor; and
- classify (block **29)** the biometric signal into one of different classes associated with the one or more behavioural states and/or transitions among awake (W), Drowsiness (D), and Sleep (S) phases based on the threshold Th computed therefor.

It is noted that, here, the sensing unit **2** is configured to:
- provide a physical interface between the sensors **3, 4** and the electronic computing resources **10;**
- extract physiological data or variables thereof from the received biometric signals; and
- transfer the physiological data to the electronic processing resources **10,** which then are configured to load and execute the software **6** to output data to be presented to an end user as local and/or remote feedback through the feedback unit.

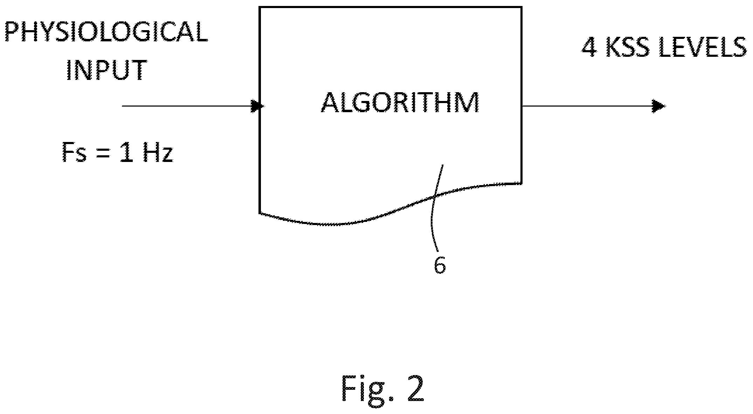

As it will be clear from the following paragraphs, with reference to Figure 2, the software **6** is configured to receive physiological data and process it to determine in real-time the drowsiness state of the person according to a reduced KSS scale, here based on four levels or classes labelled KS1, KS2, KS3 and KS4, respectively relative to the Awake state (KS1), the Drowsy state (KS2, KS3) and the Sleep state (KS4). The Applicant notes that the compression of the KSS scale from the known ten levels to the currently used four levels better reflects the behavioural state of subject (*e.g.,* KS1 is associated with the Awake state and KS4 with the Sleep state) while focusing more on the grey area describing the Drowsy state or region (here represented by classes KS2 and KS3) which is the most relevant for in-cabin monitoring applications and with particular regards to Drowsiness Monitoring Systems (DMS).

As anticipated above, each biometric signal comprises alternatively a subset of a physiological data or a variable derived from said physiological data. It is noted that the present software **6** is configured to process different physiological data, depending on the capabilities of the adopted sensing solution (either contact- or contact-less); thus, according to an aspect of the present invention, the physiological data and/or variable derived from said physiological data comprise values indicative of at least one of cardiac, respiration or eye outputs, conveniently heart rate, heart rate variability (HRV), respiration rate (RR), respiration amplitude, eye blinking or eye gazing.

In other words, the present electronic processing resources **10** are configured to process physiological data, a subset of them as well as a combination thereof.

As also shown in Figure 3, the present electronic processing resources **10** are configured to run the software 6 in a continuous loop, in particular according to the following steps.

First of all, referring to blocks **20-21,** according to a pre-processing phase, the physiological data extracted from the biometric signal is checked for validity by performing range checks on the input data, possibly exploiting Quality of Service (QoS) metrics directly provided by the sensory system; in particular, in case the quality of the physiological data is deemed insufficient to effectively quantify the drowsiness level, the physiological data is discarded and the end user is informed that no service can be provided and the next iteration of the loop is initiated. Otherwise, in case the signal quality is deemed sufficient, the next steps are carried out.

Following, referring to blocks **22-26,** according to a synthetic variable calculation step, in order to compute at least a first one synthetic quantity for and based on the received biometric signal, the software is designed to cause, when executed, the electronic processing resources **10** to become configured to compute the at least first one synthetic quantity for the received biometric signal and based on samples thereof. In particular, the synthetic quantity or variable used to classify drowsiness is computed by means of a first-in-first-out (FIFO) queue of size "n" implemented through a circular buffer. Given a sequence of n physiological measures X = {x₁, x₂, ..., xₙ}, according to an aspect of the present invention, two statistical parameters are evaluated, namely the corrected sample standard deviation for X and the mean of X. The two statistical parameters are combined into the synthetic quantity XV as the ratio of the corrected sample standard deviation of X and the mean of X. Thus, according to an aspect of the present invention, the synthetic quantity XV is a statistical dispersion index, namely a standard deviation σ².

In the case of HRV physiological data, the synthetic quantity HRVV is computed as follows: $HRVV \left(j\right) = \frac{\sqrt{\frac{{\sum }_{i = 1}^{n} {\left(HRV\left(i\right)-\frac{1}{n}{\sum }_{i = 1}^{n} HRV \left(i\right)\right)}^{2}}{n - 1}}}{\frac{1}{n} {\sum }_{i = 1}^{n} HRV \left(i\right)}$ (1)

In the case of the RR physiological data, the synthetic quantity RRV is computed as follows: $RRV \left(j\right) = \frac{\sqrt{\frac{{\sum }_{i = 1}^{n} {\left(RR\left(i\right)-\frac{1}{n}{\sum }_{i = 1}^{n} RR \left(i\right)\right)}^{2}}{n - 1}}}{\frac{1}{n} {\sum }_{i = 1}^{n} RR \left(i\right)}$ (2)

The computed synthetic quantity XV is stored in a FIFO queue of size "w."

In other words, the electronic computing resources **10** are configured, when the software 6 is being executed, to implement and make use of two FIFO queues, namely:
- a first queue relating to X and storing the latest n physiological measures (*i.e. n* physiological data), where each measure is acquired presently e.g. every second; and
- a second queue relating to the synthetic quantity XV and storing the latest w synthetic variables (*i*.*e*. w synthetic quantities XV) capturing the statistical parameters relating to X, here the ratio of the corrected standard deviation and mean.

Following, referring to blocks **27-28,** according to a calibration step, in case a given enabling condition is met (here upon initialization of the software **6** or upon a request from a user), the electronic processing resources **10** are programmed to establish the baseline status of the subject of which biometric signals are acquired. Specifically, the threshold or baseline Th is defined as function of the content of the second queue; thus, the threshold Th is a single value referring to the statistical properties of corrected standard deviation and mean of the given physiological data for a subject. Hence, the Applicant notes that the threshold Th is a snapshot of the cardio-respiratory state of a subject at a given time. Given the established threshold Th, the following processing step allows the electronic processing resources **10** to analyse the modification of the behaviour of cardio-respiratory state of the subject with respect to the established threshold Th; it is furthermore noted that, according to an aspect of the present invention, as the baseline cardio-respiratory activity may change over time, calibration may be repeated when deemed necessary, either by the electronic processing resources **10** or by the user.

Following, referring to block 29, according to a processing step, the drowsiness level of a subject, defined as a drowsiness index DOD, is computed. In particular, in order to classify the biometric signal into one of different classes associated with the one or more behavioural states and/or transitions among awake (W), Drowsiness (D), and Sleep (S) phases based on threshold computed therefor, the software is designed to cause, when executed, the electronic processing resources **10** to become configured to:
- compute (block **29)** the drowsiness index DOD on the basis of the at least a first one synthetic quantity and the computed threshold; and
- classify (block **29)** the received biometric signal into one of different classes based on the computed drowsiness index DOD.

It is noted that the number of times that in an observation windows of size w, which is the size of the second queue, the synthetic variable XV is lower than the threshold Th determined in the calibration step. As the synthetic variable XV captures the corrected standard deviation and mean of the given physiological data, the present processing step accounts for the situations in which the cardio-respiratory activity is transitioning from a state of high variability (namely, the threshold Th) to a state of lower variability.

Finally, referring to block 30, according to a feedback step, based on the DOD value, the class according to the KSS scale is determined, *i.e.* a rKSS level is determined. In particular, in order to classify the received biometric signal into one of different classes based on the computed drowsiness index, the software is designed to cause, when executed, the electronic processing resources **10** to become configured to:
- determine that, if the drowsiness index is lower than or equal to a predetermined number w divided by two, the received biometric signal is classified in a first class KS1 indicative of the awake (W) phase, *i.e.* rKSS level is equal to one;
- determine that, if the drowsiness index is higher than the predetermined number w divided by two and is lower than or equal to the predetermined number w divided by two and added to a first parameter p, the received biometric signal is classified in a second class KS2 indicative of the drowsy (D) phase *i.e.* rKSS level is equal to two;
- determine that, if the drowsiness index is higher than the predetermined number w divided by two and added to the first parameter p and is lower than or equal to the predetermined number w divided by two and added to a second parameter (r), the received biometric signal is classified in a third class KS3 indicative of the drowsy (D) phase, *i.e.* rKSS level is equal to three; and
- determine that, if the drowsiness index is higher than the predetermined number w divided by two and added to the second parameter (r), the received biometric signal is classified in a fourth class KS4 indicative of the sleep (S) phase, *i.e.* rKSS level is equal to four.

The first and second parameter p, r are constant values function of the sensitivity and the specificity of the electronic processing resources **10.**

In the context of the development of the present software **6** in particular for sleep prediction, it is important to note the following in relation to the classification and the following feedback:
- True Positive (TP) is a condition when the subject under test does fall asleep and the electronic processing resources **10** predict the event;
- True Negative (TN) is a condition when the subject under test does not fall asleep and the electronic processing resources **10** predict the event;
- False Negative (FN) is a condition when the subject under test does fall asleep and the electronic processing resources **10** does not predict the event; and
- False Positive (FP) is a condition when the subject under test does not fall asleep and the electronic processing resources **10** predict the subject to be falling asleep.

To summarise, it is noted that three numbers can be used as follows:
- sensitivity, or true positive rate is defined as $TPR = \frac{TP}{TP + FN}$, *i.e.* the ratio of positive predicted conditions (TP) over the actual number of positive conditions (TP+FN);
- specificity, or true negative rate is defined as $TNR = \frac{TN}{FP + TN}$, *i.e.* the ratio of negative predicted conditions (TN) over the actual number of negative conditions (FP+RN); and
- accuracy is defined as $ACC = \frac{TP + TN}{TP + TN + FP + FN}$,*i.e*. the radio between the correctly predicted conditions and the actual conditions.

Therefore, according to the present invention, the electronic processing resources 10 are programmed to recognise that the variability, as defined by the statistical parameters, the corrected standard deviation of X and the mean of X, of the physiological data changes over time with respect to an established baseline condition, *i.e.* the threshold Th. A reduced variability means increased fatigue according to the reduced KSS previously defined.

Upon defining the rKSS and, thus, the class associated with the biometric signal, the drowsiness index DOD is set to zero and a new loop is initiated.

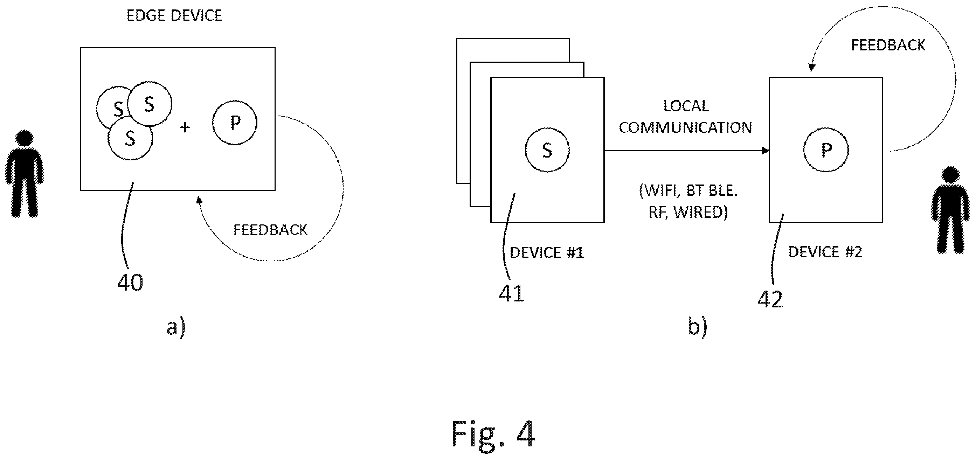

The present invention can be implemented in different architectural structures, thereby expanding the possibility of using the present invention in different contexts and applications. Examples of architectural implementations of the present invention are shown in Figures 4-9 and they will be described briefly in the following.

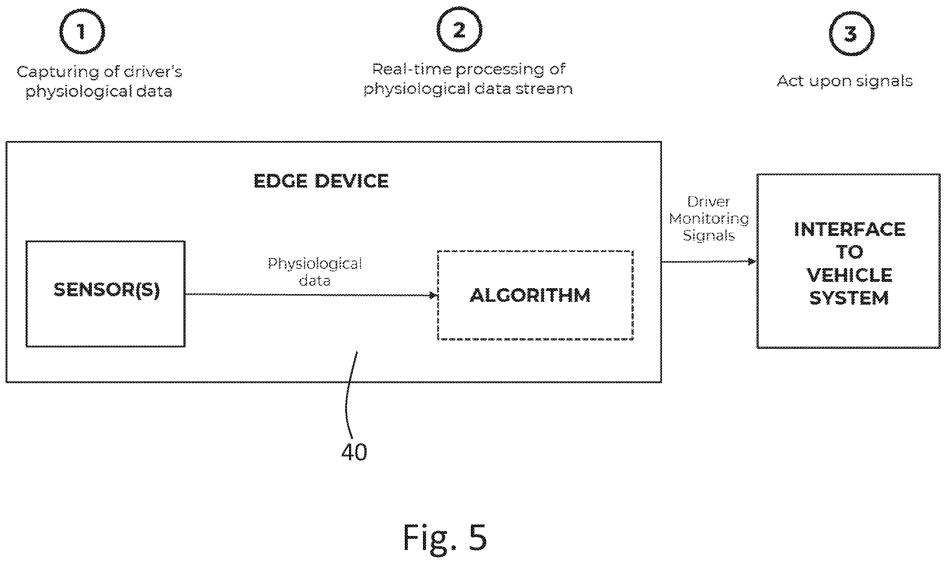

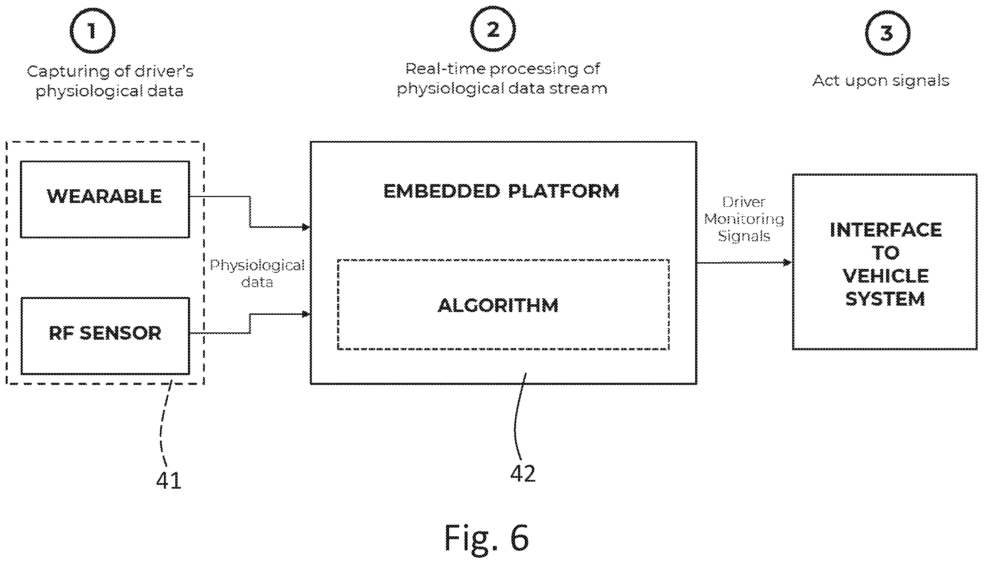

From the architectural viewpoint, both integrated and distributed solutions can be implemented, as also shown in Figures 4-6. In particular, with reference to Figure 4a) and Figure 5, showing an integrated solution, an edge device **40** is configured to fully integrate different sensors, *i.e.* integrates the sensory system of system **1,** and the processing unit **5** of the electronic processing resources **10;** furthermore, in this case, the feedback outputted by the electronic processing resources **10** is locally generated. On the other hand, with reference to Figure 4b) and Figure 6, showing a distributed solution, sensors of a first device **41** are configured to locally communicate, *e.g*. wirelessly through Wi-Fi, Blueetooth, RF signals or through a wired connection, and to transmit data to the processing unit 5 which is housed in a second device **42;** the processing unit **5** is also configured to generate the feedback.

With reference to the integrates solution shown in Figures 4a) and 5, the Applicant has carried out an experimental validation of the integrated architecture using, as edge device **40,** a modern smartwatch, being able to:
- acquire biometric signals and, thus, the related physiological data;
- run the software **6** to output a feedback to an end user; and
- warn the end user, *e.g.* a driver, by generating relevant notifications, for example in the form of haptic signals and/or visual signals, the latter generating visual notification to be shown on the screen of the smartwatch.

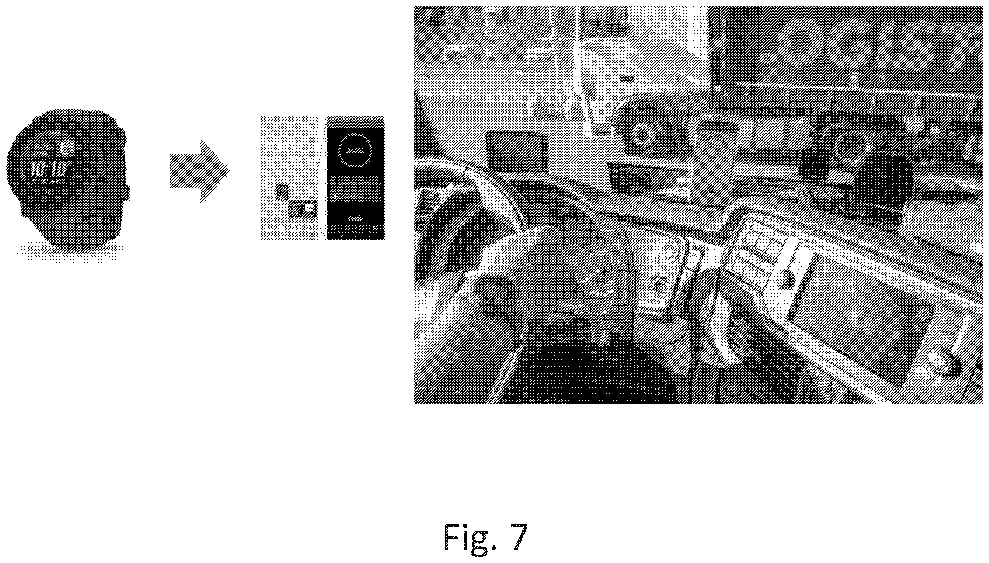

With reference to the distributed solution shown in Figures 4b) and 6, it is noted that the present invention can be applied both to short-range communication and long-range communication, as also shown in Figures 7-9.

In particular, considering a short-range communication, the end user and the system 1 are in close vicinity as, for instance, the in-cabin automotive applications or a monitoring room in a health centre; on the other hand, in the case of long-range communication, it is possible to enable new services to an end user as well as new IoT, Internet-of-Things, applications.

Referring to Figure 6, the Applicant has carried out an experimental validation of the distributed architecture on a fleet of heavy-duty trucks and involving professional drivers; with reference to Figure 7, the devices **41, 42** are respectively a smartwatch and a smartphone connected with each other through a wireless connection. The device **41** is configured to acquire the biometric signals and, thus, the physiological data and transmit them to the device **42** for the processing according to the steps described in the previous paragraphs. In particular, the device **42** is configured to run the software **6** in real-time and to communicate with a remote centre, external to both device **41, 42** to provide a feedback to the end user. In this case, the interaction with the driver is performed in two ways:
1) through the smartphone, for example through a dedicated application installed therein; and/or
2) through the infotelematic system connected to the remote centre.

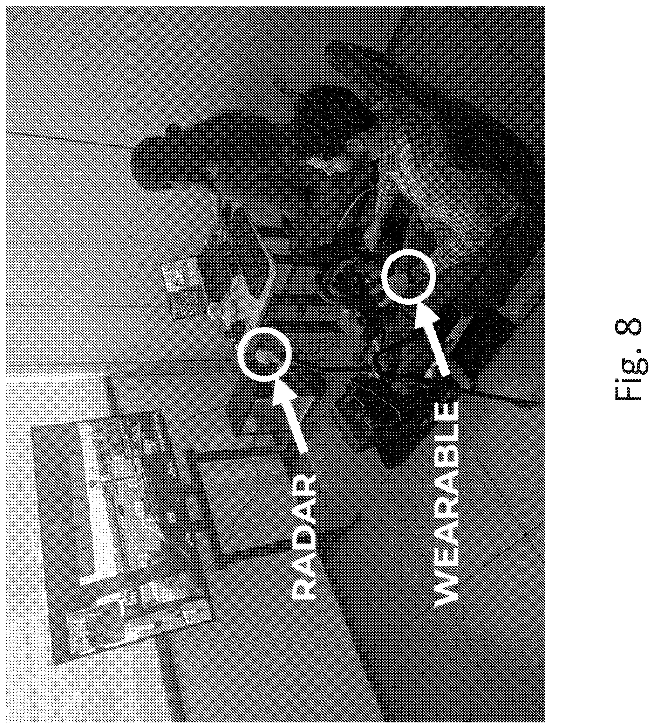

Referring to Figure 8, the Applicant has carried out a further experimental validation of the distributed architecture on a static vehicle simulator; specifically, in the case of Figure 8, two different sensors, here device **41** and in particular a wearable device such as a smartwatch, are connected wirelessly to an embedded platform, here device **42** and in particular a RADAR sensor.

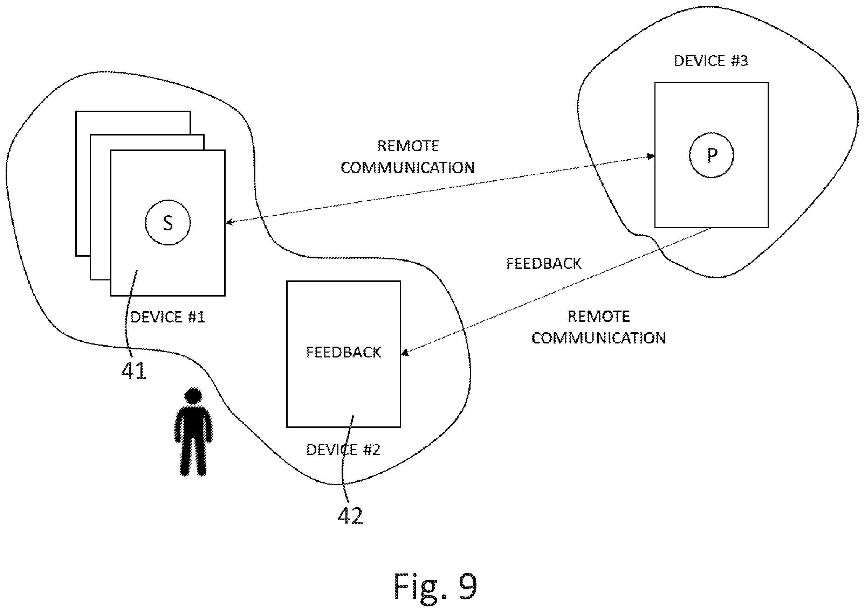

A further architectural implementation of the present invention is shown in Figure 9, wherein remote communication is envisaged and established between different devices, where sensing, processing and generating feedback are independently implemented.

The present invention has several advantages.

In particular, the present solution allows to precisely predict the sleep onset to the large variety of systems where the full PPG signal is either not accessible or available without a satisfactory quality, specifically, according to the present invention, few selected biometric parameters, either acquired from the sensors or derived from them, are used.

Furthermore, the present solution implements a statistical method in order to develop a reliable and automatic method to predict the sleep onset, thereby compensating for the relatively poor quality of the acquired/derived biometric signals.

In addition, the present invention allows to use lower quality signals and not well-defined acquisition parameters (*e.g*. sampling frequency, resolution, noise rejection, artifacts, etcetera), thereby broadening the exploitation of the present invention in a much wider range of applications and use cases.

## Claims

1. A software storable in, and executable by, electronic processing resources **(10)** and designed to cause, when executed, the electronic processing resources **(10)** to become configured to real-time predict one or more behavioural states and/or transitions among Awake, W, Drowsiness, D, and Sleep, S, phases of a subject;
the software is designed to cause, when executed, the electronic processing resources **(10)** to become configured to:
- receive **(20)** a biometric signal of a subject from a sensory system **(3, 4)** through a sensing unit **(2)** in communication with the electronic processing resources **(10);**
- process **(21-29)** the received biometric signal to classify it into one of different classes associated with the one or more behavioural states and/or transitions among Awake, W, Drowsiness, D, and Sleep, S, phases of a subject; and
- detect and/or predict **(30)** a behavioural state and/or a transition among awake, W, Drowsiness, D, and Sleep, S, phases of the subject based on the classified biometric signal,
the sensory system comprising a wearable sensor **(3)** and/or a contactless sensor **(4)** each configured to output respective biometric signals,
each biometric signal comprises alternatively a subset of a physiological data or a variable derived from said physiological data, the physiological data and/or variable derived from said physiological data comprising values indicative of at least one of cardiac, respiration or eye outputs, heart rate, heart rate variability, HRV, respiration rate, RR, respiration amplitude, eye blinking or eye gazing,
the software is configured to cause, when executed, the electronic processing resources **(10)** to become configured to:
- compute **(24)** at least a first one synthetic quantity for and based on the received biometric signal;
- compute **(29)** at least one threshold for the received biometric signal based on the at least first one synthetic quantity computed therefor; and
- classify **(29)** the biometric signal into one of different classes associated with the one or more behavioural states and/or transitions among awake, W, Drowsiness, D, and Sleep, S, phases based on the threshold computed therefor,
wherein, in order to classify **(29)** the biometric signal into one of different classes associated with the one or more behavioural states and/or transitions among awake, W, Drowsiness, D, and Sleep, S, phases based on the threshold computed therefor, the software is designed to cause, when executed, the electronic processing resources **(10)** to become configured to:
- compute (29) a drowsiness index, DOD, on the basis of the at least a first one synthetic quantity and the computed threshold; and
- classify (29) the received biometric signal into one of different classes based on the computed drowsiness index, DOD,
and wherein, in order to classify (29) the received biometric signal into one of different classes based on the computed drowsiness index, the software is designed to cause, when executed, the electronic processing resources (10) to become configured to:
- determine that, if the drowsiness index, DOD, is lower than or equal to a predetermined number, w, divided by two, the received biometric signal is classified in a first class, KS1, indicative of the awake, W, phase;
- determine that, if the drowsiness index, DOD, is higher than the predetermined number, w, divided by two and is lower than or equal to the predetermined number, w, divided by two and added to a first parameter, p, the received biometric signal is classified in a second class, KS2, indicative of the drowsiness, D, phase;
- determine that, if the drowsiness index, DOD, is higher than the predetermined number, w, divided by two and added to the first parameter, p, and is lower than or equal to the predetermined number, w, divided by two and added to a second parameter , r, the received biometric signal is classified in a third class, KS3, indicative of the drowsiness, D, phase; and
- determine that, if the drowsiness index, DOD, is higher than the predetermined number, w, divided by two and added to the second parameter, r, the received biometric signal is classified in a fourth class, KS4, indicative of the sleep, S, phase,
wherein the first and second parameter, p , r, are constant values function of the sensitivity and the specificity of the electronic processing resources (10),
wherein, in order to compute (24) at least a first one synthetic quantity for and based on the received biometric signal, the software is designed to cause, when executed, the electronic processing resources (10) to become configured to compute the at least first one synthetic quantity for the received biometric signal and based on samples thereof,
wherein the synthetic quantity is a statistical dispersion index.

2. Electronic processing resources (10) configured to real-time predict one or more behavioural states and/or transitions among awake, W, Drowsiness, D, and Sleep, S, phases of a subject; the electronic processing resources (10) being configured to store, load and execute a software according to claim 1.

## Patentansprüche

1. Software, die in elektronischen Verarbeitungsressourcen **(10)** gespeichert und von diesen ausgeführt werden kann und so ausgelegt ist, dass sie bei ihrer Ausführung bewirkt, dass die elektronischen Verarbeitungsressourcen **(10)** so konfiguriert werden, dass sie einen oder mehrere Verhaltenszustände und/oder Übergänge zwischen den "Wach-" (Awake), "W", "Schläfrigkeits-" (Drowsiness), "D", und "Schlaf-" (Sleep), "S", -Phasen einer Person in Echtzeit vorhersagen;
die Software ist so ausgelegt, dass sie bei ihrer Ausführung bewirkt, dass die elektronischen Verarbeitungsressourcen **(10)** zu Folgendem konfiguriert werden:
- Empfangen **(20)** eines biometrischen Signals einer Person von einem Sensorsystem **(3, 4)** über eine Erfassungseinheit **(2)** in Kommunikation mit den elektronischen Verarbeitungsressourcen **(10);**
- Verarbeiten **(21-29)** des empfangenen biometrischen Signals, um es in eine der verschiedenen Klassen einzustufen, die mit dem einen oder den mehreren Verhaltenszuständen und/oder Übergängen zwischen den Wach-, W, Schläfrigkeits-, D, und Schlaf-, S, -Phasen einer Person assoziiert sind; und
- Erkennen und/oder Vorherzusagen (**30**) eines Verhaltenszustands und/oder eines Übergangs zwischen den Wach-, W, Schläfrigkeits-, D, und Schlaf-, S, - Phasen der Person auf der Grundlage des eingestuften biometrischen Signals,
wobei das Sensorsystem einen tragbaren Sensor (**3**) und/oder einen berührungslosen Sensor (**4**) umfasst, die jeweils so konfiguriert sind, dass sie jeweilige biometrische Signale ausgeben,
jedes biometrische Signal alternativ einen Teilsatz physiologischer Daten oder eine von diesen physiologischen Daten abgeleitete Variable umfasst, wobei die physiologischen Daten und/oder die von diesen physiologischen Daten abgeleitete Variable Werte umfassen, die auf mindestens eines der folgenden Elemente hinweisen: Herz-, Atem- oder Augenausgangswerte, Herzfrequenz, Herzfrequenzvariabilität, HFV, Atemfrequenz, AF, Atemamplitude, Blinzeln oder Blickrichtung der Augen,
wobei die Software ferner so konfiguriert ist, dass sie bei ihrer Ausführung bewirkt, dass die elektronischen Verarbeitungsressourcen (**10**) zu Folgendem konfiguriert werden:
- Berechnen (**24**) mindestens einer ersten synthetischen Größe für und auf der Grundlage des empfangenen biometrischen Signals;
- Berechnen (**29**) mindestens eines Schwellenwerts für das empfangene biometrische Signal auf der Grundlage der mindestens einen ersten dafür berechneten synthetischen Größe; und
- Einstufen (**29**) des biometrischen Signals in eine der verschiedenen Klassen, die mit dem einen oder den mehreren Verhaltenszuständen und/oder Übergängen zwischen den Wach-, W, Schläfrigkeits-, D, und Schlaf-, S, -Phasen assoziiert sind, auf der Grundlage des dafür berechneten Schwellenwerts,
wobei die Software, um das biometrische Signal in eine der verschiedenen Klassen, die mit dem einen oder den mehreren Verhaltenszuständen und/oder Übergängen zwischen den Wach-, W, Schläfrigkeits-, D, und Schlaf-, S, -Phasen assoziiert sind, auf der Grundlage des dafür berechneten Schwellenwerts einzustufen (**29**), so ausgelegt ist, dass sie bei ihrer Ausführung bewirkt, dass die elektronischen Verarbeitungsressourcen (**10**) zu Folgendem konfiguriert werden:
- Berechnen (**29**) eines Schläfrigkeitsindex, DOD, auf der Grundlage von der mindestens einen ersten synthetischen Größe und dem berechneten Schwellenwert; und
- Einstufen (**29**) des empfangenen biometrischen Signals in eine von verschiedenen Klassen auf der Grundlage des berechneten Schläfrigkeitsindex, DOD,
und wobei die Software, um das empfangene biometrische Signal in eine von verschiedenen Klassen auf der Grundlage des berechneten Schläfrigkeitsindex einzustufen (**29**), so ausgelegt ist, dass sie bei ihrer Ausführung bewirkt, dass die elektronischen Verarbeitungsressourcen (**10**) zu Folgendem konfiguriert werden:
- Bestimmen, dass, wenn der Schläfrigkeitsindex, DOD, niedriger ist oder gleich einer vorgegebenen Zahl, w, geteilt durch zwei, das empfangene biometrische Signal in eine erste Klasse, KS1, eingestuft wird, die auf die Wach-, W, -Phase hinweist;
- Bestimmen, dass, wenn der Schläfrigkeitsindex, DOD, höher ist als die vorgegebene Zahl, w, geteilt durch zwei und niedriger ist als oder gleich der vorgegebenen Zahl, w, geteilt durch zwei und addiert zu einem ersten Parameter, P, das empfangene biometrische Signal in eine zweite Klasse, KS2, eingestuft wird, die auf die Schläfrigkeits-, D, - Phase hinweist;
- Bestimmen, dass, wenn der Schläfrigkeitsindex, DOD, höher ist als die vorgegebene Zahl, w, geteilt durch zwei und addiert zum ersten Parameter, P, und niedriger ist oder gleich der vorgegebenen Zahl, w, geteilt durch zwei und addiert zu einem zweiten Parameter, r, das empfangene biometrische Signal in eine dritte Klasse, KS3, eingestuft wird, die auf die Schläfrigkeits-, D, -Phase hinweist; und
- Bestimmen, dass, wenn der Schläfrigkeitsindex DOD höher ist als die vorgegebene Zahl w, geteilt durch zwei und addiert zum zweiten Parameter r, das empfangene biometrische Signal in eine vierte Klasse KS4 eingestuft wird, die auf die Schlaf-, S, -Phase hinweist, wobei der erste und der zweite Parameter p, r konstante Werte sind, die von der Sensitivität und der Spezifität der elektronischen Verarbeitungsressourcen (**10**) abhängen,
wobei die Software, um mindestens eine erste synthetische Größe für und auf Grundlage des empfangenen biometrischen Signals zu berechnen (**24**), so ausgelegt ist, dass sie bei ihrer Ausführung bewirkt, dass die elektronischen Verarbeitungsressourcen (**10**) so konfiguriert werden, dass sie die mindestens eine erste synthetische Größe für das empfangene biometrische Signal und auf Grundlage von Abtastwerten davon berechnen,
wobei die synthetische Größe ein statistischer Dispersionsindex ist.

2. Elektronische Verarbeitungsressourcen (**10**), die dazu konfiguriert sind, einen oder mehrere Verhaltenszustände und/oder Übergänge zwischen den Wach-, W, Schläfrigkeits-, D, und Schlaf-, S,-Phasen einer Person in Echtzeit vorherzusagen; wobei die elektronischen Verarbeitungsressourcen (**10**) dazu konfiguriert sind, eine Software nach Anspruch 1 zu speichern, zu laden und auszuführen.

## Revendications

1. Logiciel pouvant être stocké dans des ressources de traitement électronique (**10**) et exécuté par celles-ci, et conçu pour amener, lors de son exécution, les ressources de traitement électronique (**10**) à être configurées pour prédire en temps réel un ou plusieurs états comportementaux et/ou transitions entre les phases d'éveil W, de somnolence D et de sommeil S d'un sujet ;
le logiciel est conçu pour amener, lors de son exécution, les ressources de traitement électroniques (**10**) à être configurées pour :
- recevoir (**20**) un signal biométrique d'un sujet provenant d'un système sensoriel (**3**, **4**) par l'intermédiaire d'une unité de détection (**2**) en communication avec les ressources de traitement électroniques (**10**) ;
- traiter (**21-29**) le signal biométrique reçu afin de le classer dans l'une des différentes classes associées à un ou plusieurs états comportementaux et/ou transitions entre les phases d'éveil W, de somnolence D et de sommeil S d'un sujet ; et
- détecter et/ou prédire (**30**) un état comportemental et/ou une transition entre les phases d'éveil W, de somnolence D et de sommeil S du sujet sur la base du signal biométrique classé,
le système sensoriel comprenant un capteur portable (**3**) et/ou un capteur sans contact (**4**), chacun étant configuré pour émettre des signaux biométriques respectifs,
chaque signal biométrique comprenant, alternativement, un sous-ensemble de données physiologiques ou une variable dérivée desdites données physiologiques, les données physiologiques et/ou la variable dérivée desdites données physiologiques comprenant des valeurs indicatives d'au moins l'un des paramètres suivants : activité cardiaque, respiratoire ou oculaire, fréquence cardiaque, variabilité de la fréquence cardiaque (VFC), fréquence respiratoire (FR), amplitude respiratoire, clignement des yeux ou fixation du regard,
le logiciel est configuré pour amener, lors de son exécution, les ressources de traitement électroniques (**10**) à être configurées pour :
- calculer (**24**) au moins une première grandeur synthétique pour et sur la base du signal biométrique reçu ;
- calculer (**29**) au moins un seuil pour le signal biométrique reçu sur la base de l'au moins une première grandeur synthétique calculée à cet effet ; et
- classer (**29**) le signal biométrique dans l'une des différentes classes associées à un ou plusieurs états comportementaux et/ou transitions entre les phases d'éveil W, de somnolence D et de sommeil S, sur la base du seuil calculé à cet effet,
dans lequel, afin de classer (**29**) le signal biométrique dans l'une des différentes classes associées à un ou plusieurs états comportementaux et/ou transitions entre les phases d'éveil W, de somnolence D et de sommeil S, sur la base du seuil calculé à cet effet, le logiciel est conçu pour amener, lors de son exécution, les ressources de traitement électroniques (**10**) à être configurées pour :
- calculer (**29**) un indice de somnolence DOD, sur la base d'au moins une première grandeur synthétique et du seuil calculé ; et
- classer (**29**) le signal biométrique reçu dans l'une de différentes classes en fonction de l'indice de somnolence calculé DOD,
et dans lequel, afin de classer (**29**) le signal biométrique reçu dans l'une de différentes classes en fonction de l'indice de somnolence calculé, le logiciel est conçu pour amener, lors de son exécution, les ressources de traitement électroniques (**10**) à être configurées pour :
- déterminer que, si l'indice de somnolence DOD, est inférieur ou égal à un nombre prédéterminé w, divisé par deux, le signal biométrique reçu est classé dans une première classe KS1, indiquant la phase d'éveil W ;
- déterminer que, si l'indice de somnolence DOD, est supérieur au nombre prédéterminé w, divisé par deux, et est inférieur ou égal au nombre prédéterminé w, divisé par deux et ajouté à un premier paramètre P, le signal biométrique reçu est classé dans une deuxième classe KS2, indiquant la phase de somnolence D ;
- déterminer que, si l'indice de somnolence DOD, est supérieur au nombre prédéterminé w, divisé par deux et ajouté au premier paramètre P, et est inférieur ou égal au nombre prédéterminé w, divisé par deux et ajouté à un second paramètre r, le signal biométrique reçu est classé dans une troisième classe KS3, indiquant la phase de somnolence D ; et
- déterminer que, si l'indice de somnolence DOD, est supérieur au nombre prédéterminé w, divisé par deux et ajouté au second paramètre r, le signal biométrique reçu est classé dans une quatrième classe KS4, indicative de la phase de sommeil S, les premier et second paramètres p, r, étant des valeurs constantes fonction de la sensibilité et de la spécificité des ressources de traitement électronique (**10**),
dans lequel, afin de calculer (**24**) au moins une première grandeur synthétique pour et sur la base du signal biométrique reçu, le logiciel est conçu pour amener, lors de son exécution, les ressources de traitement électronique (**10**) à être configurées pour calculer l'au moins une première grandeur synthétique pour le signal biométrique reçu et sur la base d'échantillons de celui-ci,
la grandeur synthétique étant un indice de dispersion statistique.

2. Ressources de traitement électronique (**10**) configurées pour prédire en temps réel un ou plusieurs états comportementaux et/ou transitions entre les phases d'éveil W, de somnolence D et de sommeil S d'un sujet ; les ressources de traitement électronique (**10**) étant configurées pour stocker, charger et exécuter un logiciel selon la revendication 1.
